Europäisches Patentamt

European Patent Office

Office européen des brevets

(19)

(11) Veröffentlichungsnummer: **0 305 688**

**A1**

(12)

# EUROPÄISCHE PATENTANMELDUNG

(21) Anmeldenummer: **88111111.6**

(22) Anmeldetag: **12.07.88**

(51) Int. Cl.⁴: **C07C 33/042 , C07C 29/42**

(30) Priorität: **04.09.87 DE 3729678**

(43) Veröffentlichungstag der Anmeldung:
**08.03.89 Patentblatt 89/10**

(84) Benannte Vertragsstaaten:
**AT BE CH DE ES FR GB GR IT LI LU NL SE**

(71) Anmelder: **GAF-HüLS CHEMIE GMBH**
**Postfach 13 20**
**D-4370 Marl(DE)**

(72) Erfinder: **Westernacher, Helmut, Dr.**
**Burgstrasse 21**
**D-4358 Haltern(DE)**
Erfinder: **Aertken, Karl**
**Heidelohstrasse 13**
**D-4408 Dülmen(DE)**
Erfinder: **Stieren, Thomas**
**Marler Strasse 102**
**D-4358 Haltern(DE)**

(54) **Verfahren zur Herstellung von Propinol.**

(57) Bei der Herstellung von Propinol aus Formaldehyd und Acetylen kann Acetylen gelöst eingesetzt werden. Dabei werden im allgemeinen hochsiedende Lösemittel verwendet und zum Lösen des Acetylens hohe Drücke benötigt.

Bei Verwendung von Dimethoxymethan als Lösemittel kann Acetylen unter Tiefkühlung bei geringem Überdruck gefahrlos in ausreichenden Mengen gelöst werden, bevor die gekühlte Lösung in den Reaktor dosiert wird.

EP 0 305 688 A1

## Verfahren zur Herstellung von Propinol

Die Erfindung betrifft ein Verfahren zur Herstellung von Propinol aus Acetylen und Formaldehyd.

Propinol wird zur Synthese von Polyinen und von anderen Naturstoffen eingesetzt und dient außerdem als Korrosionsschutzmittel in der Galvanotechnik.

Bei der Herstellung von Butindiol-(1,4) aus Formaldehyd und Acetylen fällt Propinol in kleinen Mengen als Nebenprodukt an.

Bei den bekannten Verfahren zur Herstellung von Propinol geht man ebenfalls von Formaldehyd und Acetylen aus. Die Folgereaktion von Propinol mit Formaldehyd zu Butindiol-(1,4) wird dabei durch spezielle Reaktionsbedingungen unterdrückt.

In DE-PS 11 74 765 wird ein Zusatz von N-Methyl-pyrrolidon geschützt, das ein gutes Lösemittel für Acetylen ist und deshalb eine hohe Acetylenkonzentration ermöglicht. Die Reaktion von Acetylen und Formaldehyd in Gegenwart von Kupferacetylid führt hier überwiegend zu Propinol.

Die Abtrennung des hochsiedenden Lösemittels ist dabei aufwendig und kostenintensiv.

Nach DE-PS 12 84 964 können Acetylen und Formaldehyd in einem Lösemittel mit Hilfe von Kupferacetylid auf einem Träger zu Butindiol-(1,4) und Propinol umgesetzt werden. Dabei werden beide Produkte in vergleichbaren Mengen gebildet. Als Lösemittel wird auch Formaldehyddimethylacetal genannt, vorzugsweise wird jedoch Butyrolacton verwendet. Das Lösen erfolgt hier unter Kühlung, erfordert aber immer noch hohe Drücke, die ein erhebliches Sicherheitsrisiko darstellen.

Aufgabe der vorliegenden Erfindung ist es, ein Verfahren zu entwickeln, durch das Propinol aus Acetylen und Formaldehyd bei vermindertem Aufwand mit erhöhter technischer Sicherheit hergestellt werden kann.

Die Aufgabe wird erfindungsgemäß dadurch gelöst, daß man Acetylen unter Tiefkühlung bei Normaldruck oder geringem Überdruck in Dimethoxymethan löst und die gekühlte Lösung dem Reaktor zuführt.

Acetylen kann dabei beispielsweise in einem Absorber in Dimethoxymethan gelöst werden, wobei vorzugsweise Temperaturen von -10 bis -50 °C, besonders bevorzugt von -15 bis -40 °C, eingestellt werden. Der Druck beträgt dabei vorzugsweise 1 bis 5 bar, im besonderen 1,5 bis 4 bar.

Nach dem vorliegenden Verfahren kann Acetylen bei niedrigem Druck für die Reaktion eingeführt werden. Das Verfahren ist deshalb einfach und sicher zu handhaben. Es ermöglicht darüber hinaus eine großtechnische Auslegung bei vermindertem Risiko.

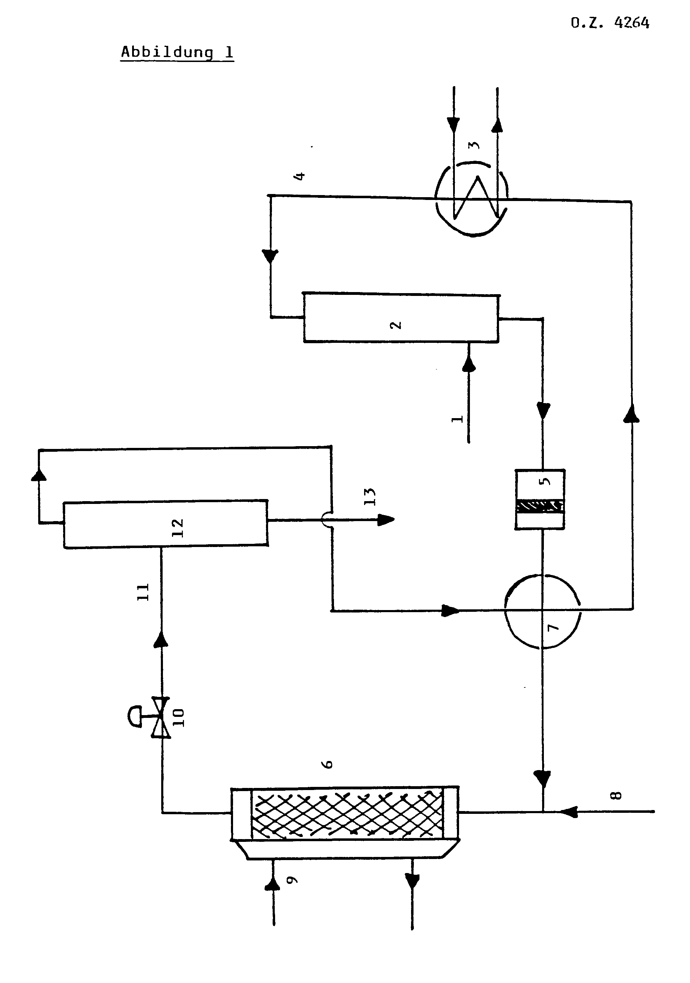

Allgemeine Verfahrensbeschreibung (vgl. Abbildung 1):

Acetylen wird über die Leitung (1) in den Absorber (2) geleitet. Als Absorber kann ein Rührkessel oder ein mit Füllkörpern gefüllter Absorptionsturm gewählt werden. Das Acetylen kann dabei im Gleich- oder auch im Gegenstrom zum Lösemittel eingespeist werden. Das Lösemittel Dimethoxymethan, das bis zu 20 % Methanol enthalten kann, vorzugsweise aber weniger als 10 % Methanol aufweist, wird im Wärmetauscher (3) abgekühlt und über die Leitung (4) in den Absorber eingetragen.

Eine mit Acetylen gesättigte Lösung - abhängig von Druck und Temperatur - wird mit einer Pumpe (5) in den Reaktor (6) gedrückt. Im Wärmetauscher (7) erfolgt zur besseren Nutzung der Energie ein Wärmetausch zwischen der Acetylenlösung und dem im Kreis geführten Dimethoxymethan. Über die Leitung (8) wird die benötigte Menge Formaldehyd zugefahren. Zum Einsatz gelangen wäßrige Formaldehydlösungen mit Formaldehydgehalten von 30 bis 80 %. Auch Formaldehydlösungen in Dimethoxymethan können eingesetzt werden.

Der Reaktor ist mit einer Katalysatorschüttung gefüllt (Festbett). Als Katalysator dient Kupferacetylid, das zum Beispiel in den in DE-PS 12 35 295 und DE-PS 10 13 279 beschriebenen Anwendungsformen vorliegen kann. Der Reaktor ist mit einer Kühl- bzw. Heizvorrichtung (9) ausgerüstet. Der Reaktor wird hydraulisch gefüllt. Mit der Druckregeleinrichtung (10) wird ein Reaktionsdruck von 10 bis 200 bar aufrechterhalten. Die Reaktionstemperatur beträgt 85 bis 150 °C.

Das Reaktionsgemisch, das im wesentlichen aus Dimethoxymethan, Methanol, Propinol, Butindiol-(1,4), Formaldehyd und Wasser besteht, wird über Leitung (11) in die Dimethoxymethan-Rückgewinnungskolonne (12) eingespeist. Das abgetrennte Dimethoxymethan/Methanol-Gemisch wird in den Prozeß zurückgeführt. Das Gemisch aus Propinol, Butindiol-(1,4), Formaldehyd und Wasser wird über die Leitung (13) einer üblichen Aufarbeitung zugeführt.

### Beispiel

In einen Rohrreaktor (Länge 1 000 mm, Innendurchmesser 23 mm), ausgerüstet mit Temperatur- und Druckmessung sowie einem Mantel zur Kühlung und Heizung, werden 500 ml Katalysator (Tabletten 1/8") eingefüllt. Der Katalysator wurde wie in DE-PS 12 35 295 beschrieben hergestellt und enthält 35 % Kupfer, 3 % $Bi_2O_3$ und 43 % Magnesiumsilikat.

In einen Rührautoklaven wird bei einer Temperatur von -16 °C und einem Druck von 2,1 bar Acetylen in Dimethoxymethan eingeleitet, wobei man eine gesättigte Lösung von Acetylen in Dimethoxymethan erzeugt.

Diese gesättigte Lösung wird mit einer Kolbenpumpe kontinuierlich in den Reaktor eingespeist (444 g/h). Zu der Lösung, die auf die Reaktionstemperatur von 115 °C aufgeheizt wird, werden 56 g/h einer 50 %igen wäßrigen Formaldehydlösung mit Hilfe einer zweiten Kolbenpumpe zudosiert.

Die entstehende Reaktionswärme wird durch Kühlung abgeführt. Der Reaktionsdruck beträgt 20 bar. Die mittlere Verweilzeit des Reaktionsgemisches im Reaktor beträgt 0,7 Stunden. Der Reaktoraustrag hat folgende Zusammensetzung:

0,3 % Formaldehyd, 6,3 % Wasser, 5,1 % Propinol, 6,3 % Butindiol-(1,4), 6,9 % Methanol, 75,1 % Dimethoxymethan

Aus 500 g/h Reaktionsgemisch werden durch Destillation Dimethoxymethan und Methanol abgetrennt und zurückgewonnen. Die Restmenge wird destillativ entsprechend DE-PS 10 02 324 aufgearbeitet. Das gewonnene Propinol ist über 99 %ig.

## Ansprüche

1. Verfahren zur Herstellung von Propinol aus Acetylen und Formaldehyd in einer Dimethoxymethanlösung an einem Kupferacetylidkatalysator, dadurch gekennzeichnet, daß man das Acetylen unter Tiefkühlung und Normaldruck oder geringem Überdruck in Dimethoxymethan löst und die gekühlte Lösung in den Reaktor einträgt.

2. Verfahren nach Anspruch 1, dadurch gekennzeichnet, daß man Acetylen bei -10 bis -50 °C und 1 bis 5 bar löst.

3. Verfahren nach Anspruch 2, dadurch gekennzeichnet, daß man Acetylen bei -15 bis -40 °C und 1,5 bis 4 bar löst.

Abbildung 1

EP 0 305 688 A1

O.Z. 4264

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 2 537 401 (GAF CORP.)  * Ansprüche 1,8,9 *  -- | 1 | C 07 C 33/042  C 07 C 29/42 |
| A | GB - A - 1.232 257 (BADISCHE ANILIN- & SODA FABRIK)  * Anspruch 1 *  -- | 1 | |
| D,A | DE - B - 1 284 964 (BADISCHE ANILIN- & SODA FABRIK)  * Anspruch *  -- | 1 | |
| A | DE - B - 1 191 364 (GENERAL ANI-LINE & FILM CORPORATION)  * Anspruch 1 *  -- | 1 | |
| D,A | DE - B - 1 174 765 (ALLIED CHEMI-CAL CORPORATION)  * Anspruch 1 *  -- | 1 | RECHERCHIERTE SACHGEBIETE (Int. Cl.4)  C 07 C 33/00  C 07 C 29/00 |
| A | GB - A - 676 601 (GENERAL ANILINE & FILM CORPORATION)  * Anspruch 1 *  ---- | 1 | |

Der vorliegende Recherchenbericht wurde für alle Patentansprüche erstellt.

| Recherchenort | Abschlußdatum der Recherche | Prüfer |
|---|---|---|
| WIEN | 18-11-1988 | REIF |